# EUROPÄISCHE PATENTSCHRIFT

(11) **EP 0 698 399 B1**
(45) Veröffentlichungstag und Bekanntmachung des Hinweises auf die Patenterteilung: **12.04.2000**
(21) Anmeldenummer: 95111647.4
(22) Anmeldetag: 25.07.1995
(51) Int. Cl.: A61M 3/02, A61J 1/16

(54) **Mobile Vorrichtung zum Einbringen einer Flüssigkeit in eine Körperhöhle**
Mobile device for introducing a liquid into a body cavity of a patient
Dispositif portable destiné à introduire un liquide dans une cavité du corps d'un patient

(30) Priorität: 23.08.1994 DE 9413529 U
(43) Veröffentlichungstag der Anmeldung: 28.02.1996
(73) Patentinhaber: Marcegaglia, Werner, D-58285 Gevelsberg (DE)
(72) Erfinder: Marcegaglia, Werner, D-58285 Gevelsberg (DE)
(74) Vertreter: Dörner, Lothar, Dipl.-Ing.

(56) Entgegenhaltungen:
- FR-A- 684 105
- US-A- 1 647 210
- US-A- 1 718 369

## Beschreibung

Auf Reisen stehen Einrichtungen zur Körperhygiene, wie sie z.B. ein stationäres Bidet bietet, das ist ein längliches Sitzwaschbecken, oft mit fester, nach oben gerichteter, regulierbarer Wasserbrause, für die Intimhygiene (Brockhaus-Enzyklopädie 3. Band 1987 S. 282), nicht immer zur Verfügung. Bekannt sind Irrigatoren, das sind Geräte zur Spülung von Körperhöhlen, vor allem von Darm, Scheide, Pleurahöhle, die aus einem zylindrischen Behälter mit Aufhängevorrichtung, einer Maßeinteilung und einem Schlauchansatz am tiefsten Punkt bestehen (Brockhaus Enzyklopädie 10. Band 1989 S. 644).

Der Erfindung liegt die Aufgabe zugrunde, eine Vorrichtung zu schaffen, die auch dann, wenn ein stationäres Bidet nicht zur Verfügung steht, Hygiene wie bei einem solchen Bidet ermöglicht, überdies medizinische Anwendungen. Gemäß der Erfindung wird diese Aufgabe gelöst durch eine verschließbare Reisetasche, die einen Wasserbehälter aus flexiblem Kunststoff mit einem Gewindeverschluß, mindestens eine auf den Gewindeverschluß schraubbare Bidetdusche sowie einen Behälter mit einem flüssigen Dusch- oder Bademittel enthält.

Gemäß der Erfindung sind eine Damenbidetdusche, die einen zwischen einem Duschkopf und einem Schraubanschluß ein relativ langes, gebogenes, festes Kunststoffrohr aufweist, und eine Herrenbidetdusche, die zwischen einem Duschkopf und einem Schraubkopf ein kurzes, gerades, festes Kunststoffrohr aufeist, in der Reisetasche vorgesehen. Diese Ausgestaltung der Vorrichtung trägt dem Umstand Rechnung, daß zur einwandfreien Damen-Hygiene andere Vorrichtungen benötigt werden als zur Herren-Hygiene.

Die Vorrichtung nach der Erfindung steht jederzeit zur Verfügung. Der Reisetasche wird zur Benutzung der Wasserbehälter entnommen. Sein Gewindeverschluß wird geöffnet, stattdessen die Bidetdusche aufgeschraubt und diese durch Druck auf den Wasserbehälter aus flexiblem Kunststoff wie ein stationäres Bidet benutzt. Dabei kann das flüssige Dusch- oder Bademittel dem weiteren Behälter entommen und verwendet werden.

FR-A-684 105 zeigt eine Reisedusche mit einer Reisetasche mit einem Seifenbehälter, einem Wasserbehälter und einen darauf aufschraubbaren Durchschlauch.

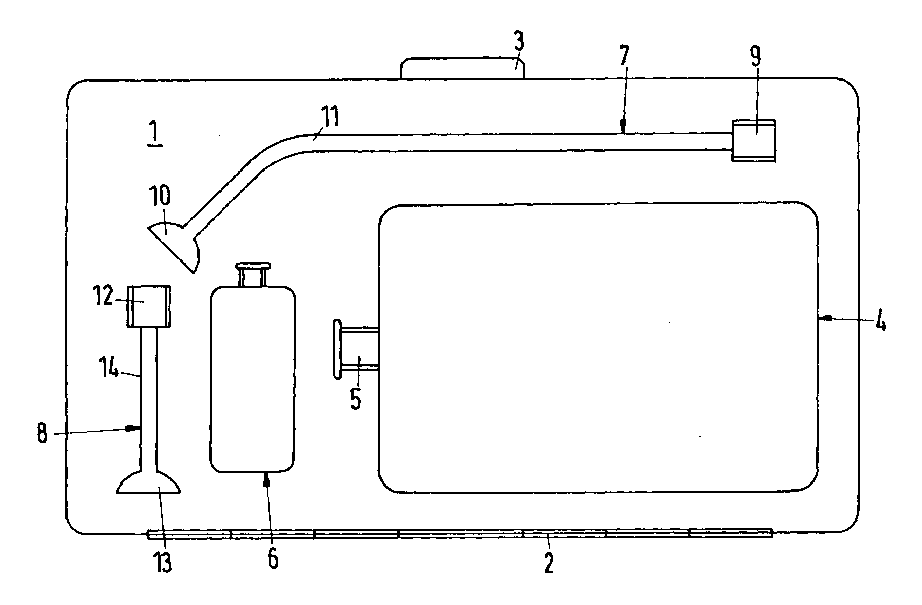

Ein Ausführungsbeispiel der Erfindung ist in der Zeichnung dargestellt und wird nachfolgend im einzelnen beschrieben. Die einzige Figur zeigt in verkleinerter schematischer Darstellung eine "durchsichtige" Reisetasche.

Eine Reisetasche 1, die im geschlossenen Zusstand dargestellt ist, weist an einer Längsseite ein Scharnier 2 und an der gegenüberliegenden Längsseite einen Verschluß 3 auf. Herkömmliche Reisetaschen sind verwendbar.

Die Reisetasche 1 enthält einen Wasserbehälter 4 aus flexiblem Kunststoff. Die Reisetasche 1 kann für diesen Wasserbehälter 4 und auch für die übrigen Teile ein Nest enthalten. Der Wasserbehälter 4 weist einen Gewindeverschluß 5 an einer Stirnseite auf. Die Reisetasche 1 enthält außerdem einen Behälter 6 für ein flüssiges Dusch- oder Bademittel, z.B. ein Duschgel.

Gemäß der Erfindung sind in der Reisetasche 1 eine Damenbidetdusche 7 und eine Herrenbidetdusche 8 vorgesehen. Die Damenbidetdusche 7 weist einen Gewindeaufsatz 9 auf. Mit diesem Gewindeaufsatz 9 wird die Damenbidetdusche 7 nach Entfernen des Gewindeverschlusses 5 auf den Wasserbehälter 4 geschraubt. Am entgegengesetzten Ende weist die Damenbidetdusche 7 einen Duschkopf 10, vorzugsweise aus Kunststoff auf. Zwischen dem Gewindeaufsatz 9 und dem Duschkopf 10 ist ein gebogenes Kunststoffrohr 11 aus hartem Material vorgesehen. Die Herrenbidetdusche 8 weist ebenfalls einen Gewindeaufsatz 12 und einen Duschkopf 13 auf. Zwischen dem Gewindeaufsatz 12 und dem Duschkopf 13 ist in diesem Fall ein kurzes gerades Kunststoffrohr 14 aus hartem Material vorgesehen.

## Patentansprüche

1. Mobile Vorrichtung zum Einbringen einer Flüssigkeit in eine Körperhöhle, mit einer verschließbaren Reisetasche (1), die einen Wasserbehälter (4) aus flexiblen Kunststoff mit einem Gewindeverschluß (5), mindestens eine auf den Gewindeverschluß (5) schraubbare Bidetdusche (7; 8) sowie einen Behälter (6) mit einem flüssigen Dusch- oder Bademittel enthält, dadurch gekennzeichnet, daß eine Damenbidetdusche (7), die zwischen einem Duschkopf (10) und einem Schraubanschluß (9) ein relativ langes, gebogenes, festes Kunststoffrohr (11) aufweist, und eine Herrenbidetdusche (8), die zwischen einem Duschkopf (13) und einem Schraubanschluß (12) ein kurzes, gerades, festes Kunststoffrohr (14) aufweist, in der Reisetasche (1) vorgesehen sind.

## Claims

1. Mobile device for introducing a liquid into a body cavity of a patient, with a lockable travelling bag (1), which comprises a water-container (4) made of flexible plastic with a threaded fastener (5), at least a bidet-shower (7; 8), screwable onto the threaded fastener (5), and a container (6) with a liquid shower or bathgel, characterized in that a lady-bidet-shower (7), which has a relatively long, curved and strong pipe made of plastic (11) between a showerhead (10) and a screw-connection (9), and man-bidet-shower (8), which has a short, straight and strong pipe made of plastic (14) between a showerhead (13) and a screw-connection (12), are provided in the travelling bag (1).

## Revendications

1. Dispositif portable destiné à introduire un liquide dans une cavité du corps d'un patient, avec une sac de voyage (1) qui ferme, qui comporte un réservoir d'eau (4) en plastique flexible avec un fermeture de filetage, au moins un douche de bidet (7; 8), vissable sur le fermeture de filetage (5), et un réservoir (6) avec un gel pour douche ou bain, caractérisé en ce que un douche de bidet pour dames (7), qui contient entre un tête de douche (10) et un raccord de vis (9) un tuyau de plastique (11) relatif long, coudé et dur, et un douche de bidet pour hommes (8), qui contient entre un tête de douche (13) et un raccord de vis (12) un tuyau de plastique (12) court, droit et dur, sont prévus dans la sac de voyage (1).
